# EUROPEAN PATENT SPECIFICATION

(11) **EP 1 928 370 B1**
(45) Date of publication and mention of the grant of the patent: **04.03.2009**
(21) Application number: 06765065.5
(22) Date of filing: 20.07.2006
(51) Int. Cl.: A61F 5/01

(54) **Joint brace**
Gelenkschiene
Support d'articulation

(30) Priority: 18.08.2005 GB 0516964
(43) Date of publication of application: 11.06.2008
(73) Proprietor: Imperial Innovations Limited, London SW7 2AZ (GB)
(72) Inventor: BULL, Anthony, Michael, James, London W3 9JA (GB); GUPTE, Chinmay, Madhukar, London, W13 9AS (GB)
(74) Representative: Korenberg, Alexander Tal
(86) International application number: PCT/GB2006/002736
(87) International publication number: WO 2007/020372

(56) References cited:
- EP-A1- 0 597 623
- US-A- 5 538 015
- US-B1- 6 322 528

## Description

This invention relates to a joint brace and in particular although not exclusively to a shoulder brace. The shoulder brace may find particular application in the treatment of anterior shoulder dislocations.

Anterior shoulder dislocation affects approximately 2% of the population, and is one of the most common musculoskeletal conditions in the young, resulting in presentation to the accident and emergency department. Most cases result from trauma resulting in forced abduction and external rotation of the shoulder. The "essential lesion" of anterior shoulder dislocation was described by Bankart in 1923 (Bankart ASB: Recurrent or habitual dislocation of the shoulder. Brit.Med.Journal 1:1132-1133, 1923), and consists of avulsion of the cartilage and capsule of the joint from the bony glenoid cavity as the humeral head dislocates.

Particularly problematic with anterior shoulder dislocation is the high rate of reoccurrence which, although decreasing with age, can be up to 50% in young active patients. There is currently a debate as to methods to prevent recurrence with treatments ranging from sling immobilisation to key-hole surgery for first time dislocators. Both of these treatments are problematic, for example because of the significant cost of key-hole surgery. Additionally, immobilisation in a sling can actually be counterproductive if it results in the seizing up of the joint. Furthermore, the inwardly rotated position of the shoulder when the arm is held in a sling is now believed to be actually counterproductive to the healing of the lesions associated with anterior shoulder dislocations.

In current treatment, after reduction of the dislocation, most patients are managed in a sling for between 3 and 6 weeks, which results in the arm being held in adduction and internal rotation. However, biomechanical and clinical research by Itoi et al (Itoi E, Hatakeyama Y, Kido T, Sato T, Minagawa H, Wakabayashi I, Kobayashi M. J: A new method of immobilization after traumatic anterior dislocation of the shoulder: a preliminary study. Shoulder Elbow Surg. 2003 12(5):413-5) has suggested that this traditional position of internal rotation may not be optimal for healing of the detached capsule and labrum. MRI studies have shown that in this position the labrum remains detached, thus exposing the glenoid rim and predisposing to recurrence. The more anatomical position of the arm in terms of reducing the capsulolabral complex appears to be in 30° of external rotation and 10-30° of abduction.

While prior art devices for maintaining the shoulder in a more anatomical position of external rotation and abduction do exist, these devices have a number of significant drawbacks. A first prior art device consists of a type of scaffolding which is applied to the whole arm and part of the upper body and is used to fix the arm and shoulder in a desired position. Another prior art device makes use of a brace attached to a user's forearm, the brace being attached to a rigid member which is worn at the user's hip. Finally, a recent prior art device includes a wedge-shaped cushion which is worn at the user's side and a lower arm brace which is attached to the cushion. All of these prior art devices fix the arm in a desired position, which, in the case of fixed external rotation, means that everyday tasks can become extremely cumbersome as the user's affected arm continuously protrudes from the side of the body. Consequently there often is a lack of compliance by the user who chooses not to wear the brace.

Furthermore, it has been recognised that a limited amount of movement is actually beneficial to the healing process and helps to prevent the joint from seizing up, which can result in a significant impairment requiring intensive treatment. All of the above-mentioned prior art braces fix the arm more or less rigidly in a given position and therefore increase the risk of the affected joints becoming stiff during treatment. Moreover, the lack of movement means that the user cannot perform simple and low-demand tasks which could otherwise be performed without affecting the healing process. This further risks reducing user compliance.

An example of prior art devices is given in US-6322528.

The present invention is described in the independent claim 1. Further, optional features of embodiments of the invention are described in the dependent claims.

A joint brace in at least some embodiments of the invention is particularly useful in, but not limited to, treatment of shoulder dislocations, is comfortable to wear and keeps interference with daily activity at a low level. The joint brace maintains a distally-adjacent joint from the joint to be treated at a certain degree of flexion thereby providing a lever for transmitting forces applied by the strap into torques or moments acting at the joint to be treated. Thus, a restoring torque or moment may be applied to the joint to be treated towards a desired position which, in the case of anterior shoulder dislocation could be a given amount of external rotation.

Advantageously, if the strap includes an elastic portion, the joint can be rotated, for example inwardly, beyond a desired angle and will experience an elastic torque which will tend to restore the arm to the desired position. Thus, the convenience and therapeutic benefit of being able move the arm, at least, to a certain extent is combined with the beneficial effect of maintaining the arm in a certain therapeutic position when the arm is relaxed. Additionally, the strap can include a tether portion or check rein which will limit the amount of movement against the elastic force to a range of movement suitable for the treatment in question.

In the case where it is desirable to maintain a shoulder in external rotation, the strap can be secured to a lateral aspect of a brace worn on the elbow, such that an outward torque can be applied to the shoulder joint. To this end, the strap could pass from the elbow brace underneath the user's armpit and across the user's chest such that movements of the shoulder joint other than rotation about the long axis of the humerus does not result in a lengthening or shortening of the strap. To this end, the elbow brace may comprise a point of attachment for the strap such that the point of attachment, the strap passing underneath the armpit, and the centre of rotation of the shoulder substantially line up.

In order to make it easier for the user to fit the elbow brace, the brace can be provided with a sliding hinge, for example by providing an axle on one part of the brace and a slot which accepts the axle on the other part of the brace. This alleviates the need to position the hinge of the elbow brace precisely aligned with the axis of rotation of the elbow joint because the axle can slide in the slot to a position where it is aligned with the axis of rotation of the elbow joint. A limiting portion of the elbow brace, which limits the amount of extension (and possibly flexion) achievable, may be fitted such that it is fixed with respect to the axle and slidable with respect to the slot, for example by providing the limiting portion with protrusions engaging with the slot.

The brace, according to at least some embodiments of the invention, can thus be used in the treatment of shoulder conditions while, at the same time, allowing substantial movement and creating a biasing force which biases the shoulder joint to a desired, therapeutic position.

Specific embodiments of the invention are now discussed by way of example only and with reference to the accompanying Figures, in which like reference numerals refer to like features and in which:
Fig. 1 shows a dorsal view of a user wearing a shoulder brace as described herein;
Fig. 2 shows a frontal view of a user wearing the joint brace;
Fig. 3 shows a portion of a strap of the joint brace shown in Figs. 1 and 2;
Fig. 4 shows a portion of an elbow brace forming part of the brace shown in Figs. 1 and 2;
Fig. 5 shows a plan view of a sliding hinge for the elbow brace;
Fig. 6 shows a cross-sectional view through the sliding joint of Fig. 5
Fig. 7 shows a further view of the elbow brace;
Fig. 8 shows the brace of Figs. 1 and 2 with an additional abduction limiting wedge; and
Fig. 9 shows a joint brace with an adduction limiting strap.

Before turning to a specific description of the embodiments, it will be useful to define certain terms describing joint movement and directions of movements with respect to the body:
- Adduction: medial movement towards the midline of the body, that is moving the elbow and arm towards the side of the body
- Abduction: lateral movement away from the midline of the body; moving the elbow and arm up and away from the side of the body
- Internal rotation: rotatory movement around a longitudinal axis at the bone between the shoulder and elbow joint (humerus) towards the centre of the body, that is turning the upper arm inward
- External rotation: rotatory movement about the same longitudinal axis away from the centre of the body, that is turning the upper ann outward
- Flexion: a decrease in the angle of the joint, for example moving the hand towards the shoulder flexes the elbow
- Extension: an increase in the angle of the joint, for example moving the hand away from the shoulder extends the elbow.

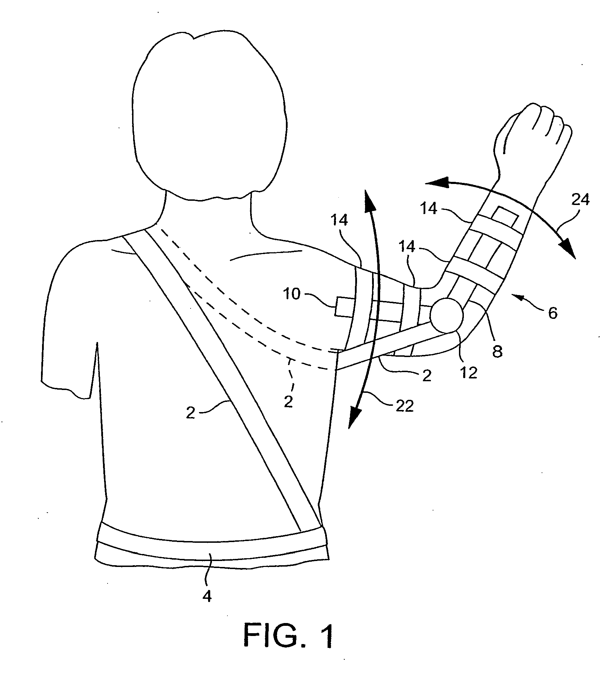

Turning now to Fig. 1, the shoulder brace includes a strap 2 secured at one end to a belt 4 worn around a user's hip or waist and at the other end to an elbow brace 6 comprising on its lateral aspect first and second rigid member 8 and 10 joined by a hinge 12 and fixed to the user's arm by straps 14 (for example nylon webbing). Abduction and adduction of the shoulder joint about an axis defined by the shoulder and the elbow (that is about the humerus) is indicated by arrow 22 and extension and flexion of the elbow is indicated by arrow 24.

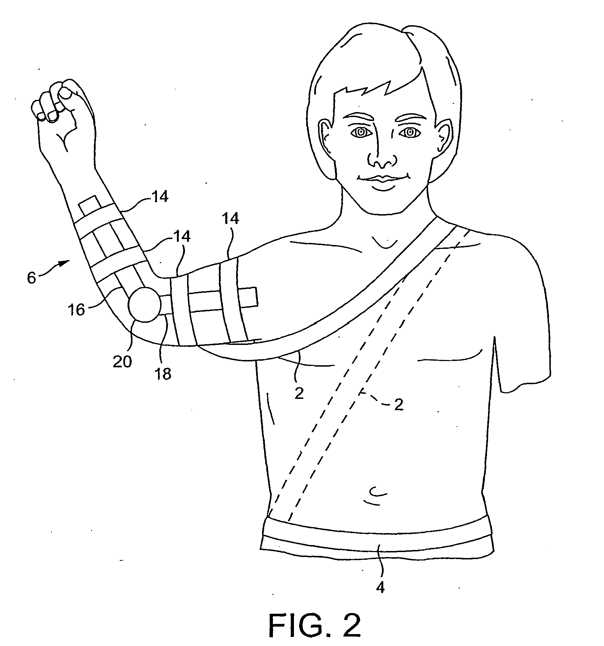

Fig. 2 shows a frontal view of the brace described above worn by a user. The medial aspect (that is on the inner side of the arm) of the elbow brace 6 includes a further first member 16, a further second member 18 and a further hinge 20 which, cooperatively with the corresponding parts on the lateral aspect of the elbow brace (that is, on the outside of the arm) forms the elbow brace. In both Figs. 1 and 2 the part of the strap 2 hidden from view by the user's body is depicted by dashed lines.

The strap 2 is attached at one end to the belt 4 on the ipsilateral (right in Fig. 1 and 2) side of the body towards the user's back and is worn across the user's back, around the contralateral (left in Fig. 1 and 2) side of the neck and across the chest and under the ipsilateral armpit to the lateral aspect of the user's arm next to the elbow where it is attached to the elbow brace 6. When the strap is worn in this way, internal rotation of the shoulder joint tends to wrap the strap around the user's elbow, thus tensioning or elongating the strap. External rotation of the shoulder results in untensioning of the strap as it is "unwrapped" from the user's elbow until the path from the point of attachment at the brace to the users armpit is shortened to such an extent that the strap is slackened.

It can thus been seen that by adjusting the length of the strap 2, a desired angle of rotation of the upper arm (and thus the shoulder) can be set, corresponding to a given path length of the strap from the armpit to the point of attachment on the elbow brace. Rotating the upper arm outwards from this desired angle results in slackening of the strap such that no force is applied by the strap to the elbow brace. Importantly, however, when the shoulder is rotated towards the body (internal rotation) beyond the desired angle, the strap 2 tensions and transmits a force, increasing with deviation from the desired angle, to the elbow brace.

Effective transmission of the above mentioned force exerted by the strap on the elbow brace to a torque opposing further internal rotation of the shoulder is achieved due to the limited extension allowed by the elbow brace. The torque is efficiently transmitted to the humerus and, thus, the shoulder joint because the angled forearm acts as a lever for transmitting the force being exerted by the strap. Without the brace maintaining a certain degree of flexion of the forearm, force transfer would be significantly less efficient since the resulting torque would be absorbed by soft tissue rotation of the upper arm and/or rotation of the forearm independent of the upper arm.

Turning now, in detail, to the construction of strap 2, the strap may be manufactured from any suitably strong material, for example nylon webbing. For added comfort of the user, the strap may be fitted with padded backing and may be sufficiently wide to avoid undue pressure on the user's skin. If the strap is manufactured entirely from an inelastic material such as nylon webbing, rotation of the shoulder inwards from the desired angle will result in large forces being applied even for small deviations from the desired angle such that the shoulder is in practice, blocked at the desired angle (apart from minor elastic behaviour due to soft tissue deformation). However, as mentioned above, it is often desirable to allow a significant amount of movement of the shoulder joint and this is seen as increasingly important in order to avoid the seizing up of the joint.

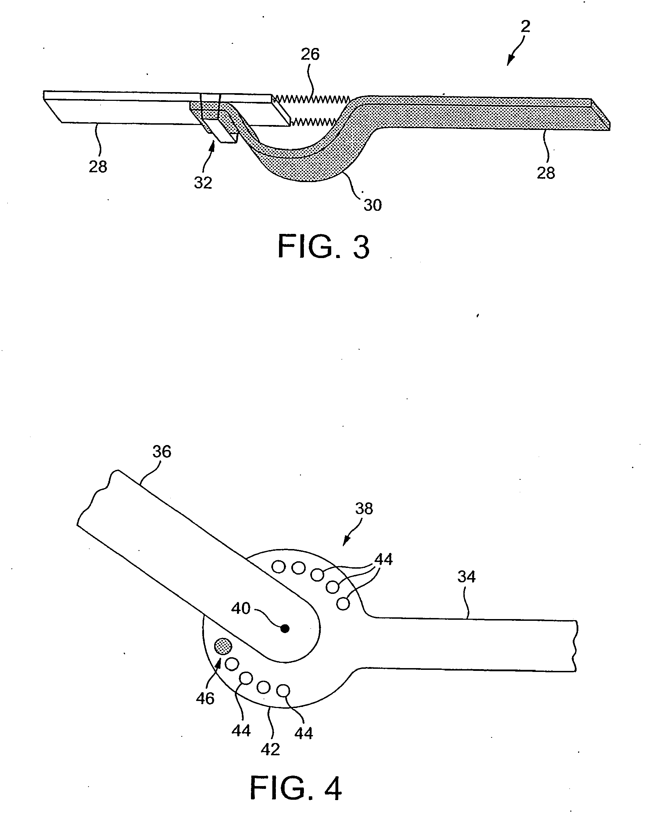

A more functional brace can be achieved by incorporating an elastic portion within the strap such that inward rotation of the shoulder beyond the desired angle results in a restoring force which increases with the deviation from the desired angle but allows a significant degree of movement (depending on the elasticity of the elastic portion). Such a functional brace allows movement beyond the desired angle, while at the same time applying a biasing force which tends to restore the shoulder joint to the desired angle of external rotation, in particular when the arm is relaxed. Fig. 3 depicts the strap 2, with an elastic proportion 26, secured in between inelastic portions 28.

In addition to providing a substantially elastic restoring torque to the shoulder joint, it may also be desirable to limit internal rotation to a maximum amount irrespective of the elasticity of elastic portion 26. To this end, a tether portion 30 can be secured to the inelastic portions 28 across the elastic portion 26, that is each end of the tether portion 30 is secured to one of the inelastic portions 28 on each side of the elastic portion 26. The tether portion 30 has a length exceeding the rest length of the elastic portion 26 and thus strap 2 can be elongated against an elastic force until the length of the elastic portion reaches the length of the tether portion, engaging the tether portion and providing a blocking force through the strap.

It is understood that the tether portion 30 may be integral with the inelastic portions 28 at one or both ends or may be secured to one or both of the inelastic portions 28 by means of, for example, stitching. An adjustable tether portion, as the one shown in Fig. 3, comprises a buckle 32 at one end thereof, allowing the length of tether portion 30, and thus the maximum elongation of the strap, to be adjusted by adjusting the buckle 32. Alternative means of adjustment could include a hook and loop fastener in place of the buckle 32.

With reference to Fig. 4, the elbow brace includes on each side thereof (medial and lateral) a first and second member 34 and 36 linked by a limiting hinge 38. The hinge 38 comprises an axle 40 for securing first member 34 to the second member 36 and a limiting member 42 defined by the first members 34 around a hole accepting the axle 40 and including a circular arrangement of holes 44 for accepting a peg 46. By securing the peg 46 within a hole 44 a corresponding desired maximum extension of the forearm can be set.

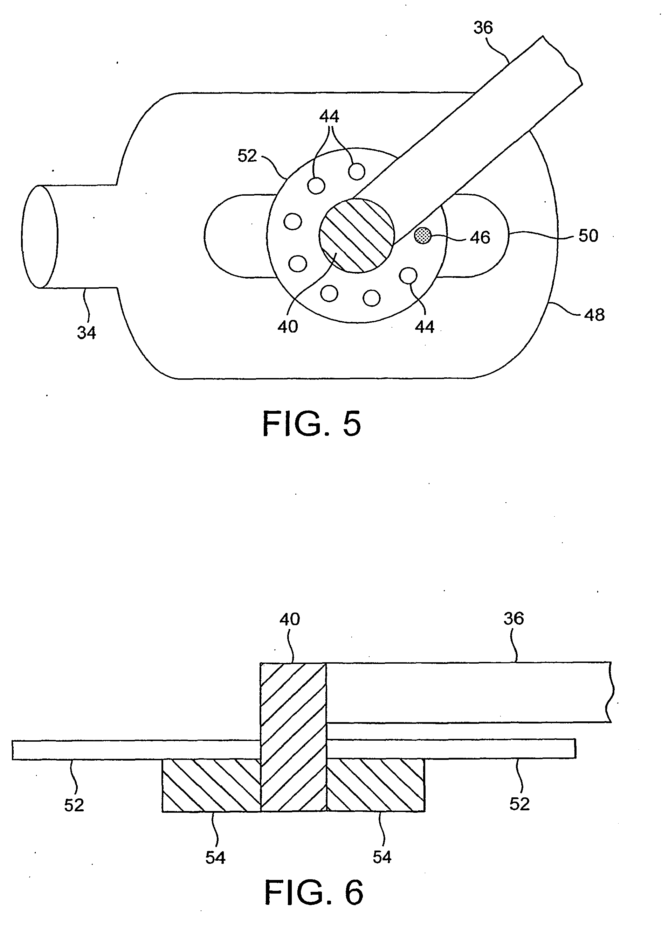

An alternative form of a limiting hinge is now described with reference to Figures 5 and 6. If the elbow brace is to be fitted by the user on a regular basis, a fixed axis hinge such as the one described with reference to Fig. 4 may be problematic in that a certain amount of locking may occur if the fixed hinge is not carefully aligned with the axis of rotation of the elbow joint. In order to increase user comfort and facilitate fitting of the elbow brace, the brace may be provided with a sliding hinge, as described below.

The first member 34 includes at one of its extremities an end portion 48 defining a slot 50. The slot 50 is arranged so as to slidably accept an axle 40 such that the second member 36 is secured both rotatably and slidingly with respect to the first members 34. The sliding hinge further includes a limiting plate 52 defining a circular arrangement of holes 44 such that a maximum degree of extension can be set by inserting peg 46 into one of the holes 44.

In order to be most effective as a means of limiting extension of the elbow brace, the limiting plate 52 must be fixed with respect to and slide together with the axle 40 with respect to the first member 34 while at the same time being rotably fixed with respect to the first member 34. This can be achieved by providing sliding members 54 secured to the limiting plate 52, as depicted in Fig. 6 which shows a cross section through the second member 36, the axle 40, the limiting plate 52 and the sliding members 54. The sliding members 54 are rigidly secured to the limiting plate 52 on either side of a hole accepting the axle 40 and arranged such that they can be inserted into the slot 50. The sliding members 54 allow the limiting plate 52 and the axle 40 to slide with respect to the first member 34 but prevent rotation of the limiting plate 52 with respect to the first member as set out above. However, satisfactory performance of the elbow brace may also be achieved using a simpler construction where the holes 44 are provided on the end portion 48 (resulting in a maximum flexion angle dependent on the position of the axle 40 inside the slot 50).

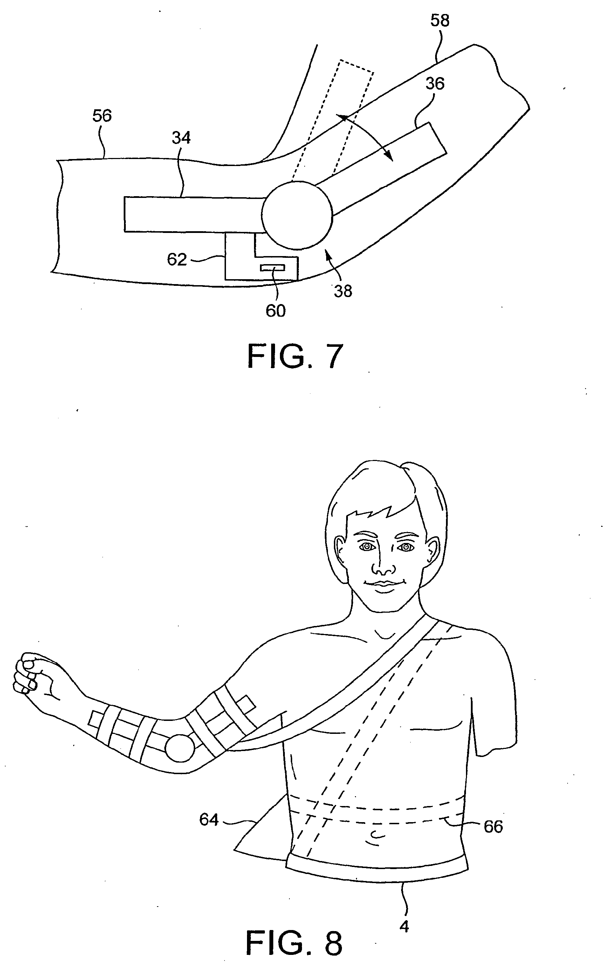

With elbow extension limited by the elbow brace as described above, any point of attachment on the elbow brace (or even anywhere on the user's forearm) is conceivable. However, a particular implementation of the elbow brace provides a point of attachment for the strap 2 which ensures that the length of the strap 2 is unaffected by movement of the shoulder joint other than internal/external rotation. This will now be described with reference to Figure 7.

With the first members 34 secured to the user's upper arm 56 and the second member 36 secured to the user's forearm 58, the hinge 38 being aligned with the centre of rotation of the elbow, a point of attachment 60 for the strap is provided on a member 62 extending distally and posteriorally towards the distal end of the upper arm. The point of attachment 60 is arranged to be close to the distal posterior end of the upper arm such that the strap is guided from the armpit along the posterior surface of the upper arm to the strap attachment point 60. This arrangement serves to minimise the influence of movements other than external or anterior rotation (for example flexion, extension, abduction, or adduction of the shoulder) such that the desired angle of rotation at which the strap exerts its restoring force is substantially independent of the overall position of the arm.

While in the description above the first member 34 has been described as being worn on the upper arm and the second member 36 has been described as being worn on the lower arm, the reverse situation is equally envisaged. In case that the second member 36 is worn on the upper arm, and the member 62 extends from the second member 36 rather than from the first member 34

The shoulder brace described above is designed to limit only internal rotation (and possibly external rotation - see below) of the shoulder without affecting any other degree of freedom. In the treatment of many conditions of the shoulder, for example in the treatment of anterior shoulder dislocation, it is desirable also to also limit adduction of the shoulder, that is to maintain a minimum angle between the arm and the side of the body. To this end, an optional abduction limiting wedge 64 can be added, as shown in Figure 8. The wedge 64 can be secured to the belt 4 at its lower extremity and to an additional waistband 66 at its upper extremity.

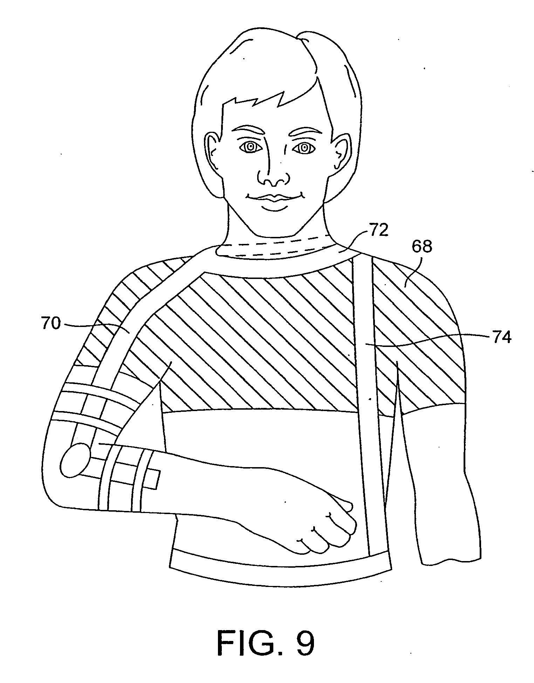

As an alternative to use of the wedge 64 for limiting adduction of the upper arm, adduction can also be limited by providing a further strap running over the top of the shoulder. Figure 9 illustrates an alternative embodiment of the shoulder brace, where the belt 4 may be replaced with a harness or vest 68 worn on the upper body. A further strap 70 can be attached between the vest 68 and the elbow brace 6, running across the shoulder, such that the further strap 70 is tensioned when the arm is adducted beyond a desired angle. The vest 68 may contain loops or guides (on the torso, arm or both) for guiding the further strap 70 (and any other strap, for example the strap 2, which may be used with the vest 68). Guides on the lateral aspect of the arm may serve to prevent twisting of the further strap 70 around the arm. The further strap 70 is secured to the vest by a loop 72 of, for example, nylon webbing, around the user's neck, which loop will tend to dissipate forces transmitted through the further strap 70. The loop may further comprise a suitably rigid member (not shown) which prevents tightening of the loop around the neck as forces are applied by the further strap 70.

Optionally, a further securing strap 74 can be attached between the loop 72 and the belt 4 on the contralateral side of the body such that forces applied by the further strap 70 are transmitted to the point of attachment of the securing strap 74 to the belt 4. As an alternative to the vest, adduction control may be implemented analogously without using the vest 68 with the further strap 70, the loop 72 and the securing strap 74 attached to the belt 4.

Although the rotation limiting strap described above is not shown in Figure 9, it will be understood that the two functionalities may be combined in a single brace. The vest 68 may provide for an alternative point of attachment on the body for the strap 2, which can be secured to the harness, for example at a point on the user's chest close to the relevant armpit. Of course, other means of attaching the straps (2 and 70) to the body are equally envisaged, including a chest strap, the above-mentioned belt or harness, as well as a combination of these.

The shoulder brace, as described above, provides a restoring force biasing the shoulder joint towards a desired angle of external rotation when the shoulder joint is rotated internally beyond the desired angle. It may also be desirable to provide for a further biasing force which tends to restore the shoulder joint to the desired angle or position when the joint is rotated externally beyond the desired position. This can be achieved by fitting a further strap in a similar position to the strap 2 but secured to the medial aspect of the elbow brace and the back of harness 68 in Figure 9.

In the embodiment described above with reference to Figs. 1 and 2, the further strap would extend from the medial aspect of the elbow brace, underneath the armpit to the user's back and over the contralateral side of the neck to the ipsilateral corner of the belt 4 towards the front of the body. The choice of an elastic or inelastic strap and the length of the strap are analogous to the considerations for the strap 2 and the same considerations apply as regarding the elbow brace and the attachment of the strap thereto.

Using a combination of one, two, or three straps attached between a securing portion (belt, chest, strap or harness) on the user's body and the elbow brace 6, a functional bracing of the shoulder joint can thus be achieved which creates forces opposing one or more of internal rotation, external rotation and adduction. It is understood that in all of these combinations one or all of the straps may comprise an elastic and/or tether portion, as described above. The said portion may be located in any suitable position, as will be apparent to the skilled person. For example, by placing the said portion on the user's arm, movement of the straps on the user's torso may be minimised.

It will be understood by the skilled person that the joint brace described above is not limited to orthopaedic or therapeutic uses, but can also be used as a preventative measure, for example in sport. One mechanism by which a brace of the kind described above can be useful when worn during sporting activities is that the elastic force provided by the brace may provide additional proprioceptive feedback which could give the sportsman an improved estimate of the position of the joint. It is clear that this can be done with any combination of straps, as set out above.

Furthermore, the skilled person will understand that the brace described above is not limited for use as a shoulder brace. It may be applied to any joint which has a rotatory degree of freedom, for example the hip joint. In this case the above-described elbow brace would be replaced by an analogous knee brace, limiting extension of the knee in order to provide a lever for converting the force exerted by the strap into a torque acting on the joint. For example, such a hip brace may be used in the treatment of certain conditions such as hip dysplasia occurring in newly born infants where a certain degree of rotation of the hip joint is at the present maintained by applying a cast.

Many modifications, alterations or juxtapositions of the features described above in relation to the exemplary embodiments will be apparent to the skilled person. Accordingly, the above exemplary embodiments are illustrative only of the invention and are not limiting on the invention which is defined in the appended claims.

## Claims

1. A joint brace adapted to control, when worn by a user, rotation of a first joint about an axis defined by the first joint and a second joint distally adjacent to the first joint, the brace including:
a first securing portion (4) adapted to be worn by the user;
a second securing portion (6) to be worn by the user distally of the first joint; and
a first strap (2), secured to the first (4) and second (6) securing portions, the first strap (2) being arranged to apply a force to the second securing portion (6) such that a restoring torque towards a desired angle is applied to the first joint when the first joint is rotated about the axis beyond the desired angle;
**characterised in that** the second securing portion (6) includes a hinged brace limiting extension of the second joint.

2. A joint brace as claimed in claim 1, the hinged brace including a first (8) and a second (10) rigid member and a sliding hinge, including an axle (40) slidably and rotably held in a slot (50), linking the said members, one of the said members defining the axle (40) and the other of the said members defining the slot (50), the slot being arranged to accept the axle.

3. A joint brace as claimed in claim 2, the hinge including a limiting portion arranged to limit the angular range of the hinge, the limiting portion (52) being disposed between the first and second members and including protrusions (54) engaging with the slot to prevent rotation of the limiting portion with respect to the said other member.

4. A joint brace as claimed in claim 2 or claim 3, the hinged brace including a biasing portion applying a flexing torque to the hinged brace when the second joint is extended beyond a desired position.

5. A joint brace as claimed in any one of the preceding claims in which the strap includes an elastic portion (26) such that, in use, the joint can be rotated substantially beyond the desired angle, the restoring torque being a substantially elastic torque.

6. A joint brace as claimed in claim 5, in which the strap includes a tether portion (30) such that, in use, the joint can be rotated substantially beyond the desired angle up to an angle of maximum rotation.

7. A joint brace as claimed in claim 6 in which the tether portion (30) includes an inelastic portion (30), secured to the first strap adjacent to respective ends of the elastic portion (26), the elastic portion having a rest length and the tether portion (30) having a length exceeding the rest length.

8. A joint brace as claimed in any one of the preceding claims arranged for wearing such that the first joint is the user's shoulder and the second joint is the user's corresponding elbow.

9. A joint brace as claimed in claim 8, the first strap (2) being secured to a lateral aspect of the second securing portion (6), the restoring torque being an outward torque applied when the shoulder joint is rotated about the axis inwards beyond the desired angle.

10. A joint brace as claimed in claim 9, the first strap (2) passing in use from the second securing portion (6) underneath the armpit and across the user's chest, the second securing portion including a strap attachment member (60) defining a point of attachment for the first strap arranged such that the point of attachment, a portion of the strap passing underneath the armpit and the centre of the rotation of the shoulder joint substantially lie on a straight line.

11. A joint brace as claimed in claim 10, the strap attachment member (60) extending distally and posteriorly from the second securing portion proximal to the shoulder.

12. A joint brace as claimed in any one of claims 8 to 11 including a second strap secured to a medial aspect of the second securing portion (6) and arranged to apply a force to the second securing portion such that a restoring inward torque is applied to the shoulder joint when the shoulder joint is rotated about the axis outwards beyond a desired angle.

13. A joint brace as claimed in any one of claims 8 to 12, including a third strap passing, in use, from the first securing portion (4) across the top of the shoulder to the second securing portion (6), the third strap applying a restoring force to the second securing portion when the shoulder is adducted beyond a desired adduction angle.

14. A joint brace as claimed in any one of claims 8 to 12 including a wedge portion (69) attached to the first securing portion to limit adduction of the shoulder.

15. A set of components adapted to be assembled into a joint brace as claimed in any one of the preceding claims.

## Patentansprüche

1. Gelenkstütze, die eingerichtet ist, um die Drehung eines ersten Gelenks um eine Achse zu steuern, wenn sie von einem Benutzer getragen wird, wobei die Achse vom ersten Gelenk und einem zweiten Gelenk, das dem ersten Gelenk entfernt benachbart ist, definiert ist, wobei die Stütze enthält:
ein erstes Befestigungsmittel (4), das eingerichtet ist, um von einem Benutzer getragen zu werden;
ein zweites Befestigungsmittel (6), das vom Benutzer distal vom ersten Gelenk zu tragen ist; und
einen ersten Riemen (2), der am ersten (4) und zweiten (6) Befestigungsmittel befestigt ist, wobei der erste Riemen (2) angeordnet ist, um Kraft auf das zweite Befestigungsmittel (6) auszuüben, so dass ein Rückstellmoment hin zu einem gewünschten Winkel auf das erste Gelenk ausgeübt wird, wenn das erste Gelenk um die Achse über den gewünschten Winkel hinaus gedreht wird;
**dadurch gekennzeichnet, dass** das zweite Befestigungsmittel (6) eine angelenkte Stütze enthält, welche die Dehnung des zweiten Gelenks begrenzt.

2. Gelenkstütze nach Anspruch 1, wobei die angelenkte Stütze ein erstes (8) und ein zweites (10) starres Element und ein Gleitgelenkstück mit einer Achse (40) umfasst, die in einem Schlitz (50) verschiebbar und drehbar gehalten wird, wodurch die Elemente verbunden sind, wobei eines der Elemente die Achse (40) und das andere der Elemente den Schlitz (50) definiert, wobei der Schlitz angeordnet ist, um die Achse aufzunehmen.

3. Gelenkstütze nach Anspruch 2, wobei das Gelenkstück einen Begrenzungsabschnitt enthält, der angeordnet ist, um den Winkelbereich des Gelenkstücks zu begrenzen, wobei der Begrenzungsabschnitt (52) zwischen dem ersten und dem zweiten Element angeordnet ist und Vorsprünge (54) aufweist, die in den Schlitz eingreifen, um eine Drehung des Begrenzungsabschnitts in Bezug auf das andere Element zu verhindern.

4. Gelenkstütze nach Anspruch 2 oder 3, wobei die angelenkte Stütze einen Vorspannabschnitt enthält, der ein Durchbiegungsmoment auf die angelenkte Stütze ausübt, wenn das zweite Gelenk über eine gewünschte Position hinaus gedehnt wird.

5. Gelenkstütze nach einem der vorstehenden Ansprüche, wobei der Riemen einen elastischen Abschnitt (26) enthält, so dass das Gelenk, wenn in Einsatz, im Wesentlichen über den gewünschten Winkel hinaus gedreht werden kann, wobei das Rückstellmoment im Wesentlichen ein elastisches Moment ist.

6. Gelenkstütze nach Anspruch 5, wobei der Riemen ein Haltegurtmittel (30) aufweist, so dass das Gelenk, wenn in Einsatz, im Wesentlichen über den gewünschten Winkel bis zu einem maximalen Drehwinkel gedreht werden kann.

7. Gelenkstütze nach Anspruch 6, wobei das Haltegurtmittel (30) einen nicht-elastischen Abschnitt (30) aufweist, der am ersten Riemen benachbart den jeweiligen Enden des elastischen Abschnitts (26) befestigt ist, wobei der elastische Abschnitt eine Restlänge aufweist, und das Haltegurtmittel (30) eine Länge hat, die länger als die Restlänge ist.

8. Gelenkstütze nach einem der vorstehenden Ansprüche, die angeordnet ist, um so getragen zu werden, dass das erste Gelenk die Schulter des Benutzers und das zweite Gelenk der entsprechende Ellbogen des Benutzers ist.

9. Gelenkstütze nach Anspruch 8, wobei der erste Riemen (2) an einem seitlichen Punkt des zweiten Befestigungsmittels (6) befestigt ist, wobei das Rückstellmoment ein Moment nach außen ist, das angelegt wird, wenn das Schultergelenk nach innen um die Achse über den gewünschten Winkel hinaus gedreht wird.

10. Gelenkstütze nach Anspruch 9, wobei der erste Riemen (2), wenn in Verwendung, vom zweiten Befestigungsmittel (6) unterhalb der Achselhöhle und über die Brust des Benutzers verläuft, wobei das zweite Befestigungsmittel ein Riemenbefestigungselement (60) enthält, das einen Befestigungspunkt für den ersten Riemen definiert, so angeordnet, dass der Befestigungspunkt, ein Abschnitt des Riemens, der unterhalb der Achselhöhe verläuft, und das Drehzentrum des Schultergelenks im Wesentlichen auf einer geraden Linie liegen.

11. Gelenkstütze nach Anspruch 10, wobei sich das Riemenbefestigungselement (60) distal und hinter dem zweiten Befestigungsmittel nahe der Schulter befindet.

12. Gelenkstütze nach einem der Ansprüche 8 bis 11, mit einem zweiten Riemen, der an einem mittleren Punkt des zweiten Befestigungsmittels (6) befestigt und angeordnet ist, um eine Kraft auf das zweite Befestigungsmittel auszuüben, so dass ein Rückstellmoment nach innen auf das Schultergelenk ausgeübt wird, wenn das Schultergelenk nach außen um die Achse über einen gewünschten Winkel hinaus gedreht wird.

13. Gelenkstütze nach einem der Ansprüche 8 bis 12, mit einem dritten Riemen, der, wenn in Verwendung, vom ersten Befestigungsmittel (4) über den oberen Teil der Schulter zum zweiten Befestigungsmittel (6) verläuft, wobei der dritte Riemen eine Rückstellkraft auf das zweite Befestigungsmittel ausübt, wenn die Schulter über einen gewünschten Adduktionswinkel hinaus adduziert wird.

14. Gelenkstütze nach einem der Ansprüche 8 bis 12, mit einem Keilabschnitt (69), der am ersten Befestigungsmittel angebracht ist, um die Adduktion der Schulter zu begrenzen.

15. Satz von Komponenten, der eingerichtet ist, um zu einer Gelenkstütze nach einem der vorstehenden Ansprüche zusammengefügt zu werden.

## Revendications

1. Support d'articulation adapté pour contrôler, lorsqu'il est porté par un utilisateur, la rotation d'une première articulation autour d'un axe défini par la première articulation et une deuxième articulation adjacente distalement de la première articulation, le support comprenant :
une première partie de fixation (4) adaptée pour être portée par l'utilisateur ;
une deuxième partie de fixation (6) destinée à être portée par l'utilisateur distalement de la première articulation ; et
une première sangle (2), fixée aux première (4) et deuxième (6) parties de fixation, la première sangle (2) étant disposée pour appliquer une force à la deuxième partie de fixation (6) de telle sorte qu'un couple de restauration en direction d'un angle souhaité est appliqué à la première articulation lorsque la première articulation est tournée autour de l'axe au-delà de l'angle souhaité ;
**caractérisé en ce que** la deuxième partie de fixation (6) comprend un support articulé limitant l'extension de la deuxième articulation.

2. Support d'articulation selon la revendication 1, le support articulé comprenant un premier (8) et un deuxième (10) membre rigide et une charnière coulissante, comprenant un axe (40) maintenu de manière coulissante et de manière pivotante dans une encoche (50), liant lesdits membres, un desdits membres définissant l'axe (40) et l'autre desdits membres définissant l'encoche (50), l'encoche étant disposée pour accepter l'axe.

3. Support d'articulation selon la revendication 2, la charnière comprenant une partie limitante disposée pour limiter la plage angulaire de la charnière, la partie limitante (52) étant disposée entre les premier et deuxième membres et comprenant des parties saillantes (54) venant en prise avec l'encoche pour empêcher une rotation de la partie limitante par rapport audit autre membre.

4. Support d'articulation selon la revendication 2 ou la revendication 3, le support articulé comprenant une partie de déviation appliquant un couple de flexion au support articulé lorsque la deuxième articulation est déployée au-delà d'une position souhaitée.

5. Support d'articulation selon l'une quelconque des revendications précédentes, dans lequel la sangle comprend une partie élastique (26) de telle sorte que, en cours d'utilisation, l'articulation peut être tournée sensiblement au-delà de l'angle souhaité, le couple de restauration étant un couple sensiblement élastique.

6. Support d'articulation selon la revendication 5, dans lequel la sangle comprend une partie d'attache (30) de telle sorte que, en cours d'utilisation, l'articulation peut être tournée sensiblement au-delà de l'angle souhaité jusqu'à un angle de rotation maximale.

7. Support d'articulation selon la revendication 6, dans lequel la partie d'attache (30) comprend une partie non élastique (30), fixée à la première sangle adjacente aux extrémités respectives de la partie élastique (26), la partie élastique ayant une longueur au repos et la partie d'attache (30) ayant une longueur dépassant la longueur au repos.

8. Support d'articulation selon l'une quelconque des revendications précédentes, disposé pour le port de telle sorte que la première articulation est l'épaule de l'utilisateur et la deuxième articulation est le coude correspondant de l'utilisateur.

9. Support d'articulation selon la revendication 8, la première sangle (2) étant fixée à un aspect latéral de la deuxième partie de fixation (6), le couple de restauration étant un couple appliqué vers l'extérieur lorsque l'articulation de l'épaule est tournée autour de l'axe vers l'intérieur au-delà de l'angle souhaité.

10. Support d'articulation selon la revendication 9, la première sangle (2) passant en cours d'utilisation de la deuxième partie de fixation (6) en dessous de l'aisselle et à travers la poitrine de l'utilisateur, la deuxième partie de fixation comprenant un membre d'attachement de sangle (60) définissant un point d'attachement pour la première sangle disposé de telle sorte que le point d'attachement, une partie de la sangle passant en dessous de l'aisselle et le centre de la rotation de l'articulation de l'épaule se trouvent sensiblement sur une ligne droite.

11. Support d'articulation selon la revendication 10, le membre d'attachement de sangle (60) s'étendant distalement et postérieurement à partir de la deuxième partie de fixation proximale à l'épaule.

12. Support d'articulation selon l'une quelconque des revendications 8 à 11, comprenant une deuxième sangle fixée à un aspect médian de la deuxième partie de fixation (6) et disposé pour appliquer une force à la deuxième partie de fixation de telle sorte qu'un couple de restauration vers l'intérieur est appliqué à l'articulation de l'épaule lorsque l'articulation de l'épaule est tournée autour de l'axe vers l'extérieur au-delà d'un angle souhaité.

13. Support d'articulation selon l'une quelconque des revendications 8 à 12, comprenant une troisième sangle passant, en cours d'utilisation, de la première partie de fixation (4) à travers le sommet de l'épaule à la deuxième partie de fixation (6), la troisième sangle appliquant une force de restauration à la deuxième partie de fixation lorsque l'épaule est approchée au-delà d'un angle d'adduction souhaité.

14. Support d'articulation selon l'une quelconque des revendications 8 à 12 comprenant une partie biseautée (69) fixée à la première partie de fixation pour limiter l'adduction de l'épaule.

15. Ensemble de composants adaptés pour être assemblés en un support d'articulation selon l'une quelconque des revendications précédentes.
